# EUROPEAN PATENT APPLICATION

(11) **EP 3 097 906 A1**
(43) Date of publication of application: **30.11.2016**
(21) Application number: 15708591.1
(22) Date of filing: 26.01.2015
(51) Int. Cl.: A61K 9/00, A61K 31/167

(54) **PHARMACEUTICAL FORMULATION FOR ADMINISTRATION OF MEDICINES**

(30) Priority: 24.01.2014 PT 10742414
(71) Applicant: Doctor Gummy S.A., 4250-256 Porto (PT)
(72) Inventor: DE ALMEIDA SANTOS, Paulo, P-4460-347 Senhora Da Hora (PT)
(74) Representative: Ferreira, Maria Silvina
(86) International application number: PCT/IB2015/050590
(87) International publication number: WO 2015/111020

(57) **Abstract**

The present application refers to the formulation of medicines based on healthy, natural, gluten-free, lactose-free excipients without coloring agents or artificial preservatives.

## Description

### Technical Field

The present application refers to the formulation of medicines based on healthy, natural, gluten-free, lactose-free excipients without coloring agents or artificial preservatives.

### Summary

The pharmaceutical formulation of the present application facilitates the administration and the swallowing of medicines by using sweet excipients that dissolve rapidly when in contact with the oral mucosa in the mouth. The degree of dissolution varies according to the desired level and time of absorption for each active substance.

The pharmaceutical formulation allows you to mask the unpleasant taste of the active substance(s), because the healthy and natural sweets used as the basis for the introduction of those active substances have strong aromas, e.g. fruit, through the inclusion of a large amount of fruit in its composition, and are naturally sweetened, without resort to synthetic or other sugars, harmful to both humans and animals.

The present pharmaceutical formulation also facilitates the administration of medicines to children comprising mainly or only natural natural and healthy ingredients, innocuous (not harmful or dangerous to the health of humans and/or animals for whom the formulations are intended to) without the addition of harmful artificial sugars or sweeteners and/or artificial flavoring, commonly used in traditional formulations of medicines or drugs and that contribute to the onset of dental caries among children, as well as for overweight and diabetes.

The solution provided in the present application provides a healthier drug presentation, more appealing to children and elders, very easy to administrate, which until now was not possible to achieve by any of the products available on the market, presenting itself as the best solution to the main issues pointed out by the patients who have to ingest medication, which had no appropriate and comfortable solution until now, or the provided solution implies negative consequences for the patient or consumer.

### Background

Document WO 2014/163492 discloses a composition, in particular a gelatin or jelly and its use as vehicle for better swallowing the oral medication in solid or other form, this composition comprising carrageenan as gelling agent and citric acid as salivating agent, characterized in that the composition additionally comprises maltodextrin. In another embodiment, the composition comprises an agent for the uptake of calcium to adjust the activity of the Ca ion within the composition. In one embodiment, the composition comprises carrageenan in the proportion of 0,7 -1,0% by weight, citric acid in the proportion of 0,06 -0,07% by weight, maltodextrin in the proportion of 1,5% by weight, all in relation to the total weight of the composition and an amount of the agent for the uptake of calcium such that the activity of calcium ion from the composition is between 20 ppm and 80 ppm.

Document WO 2011/014960 discloses a process for obtaining an edible gum, the mentioned process comprising the provision of a sugar or sugar alcohol, water and heating it, at least, up to 80 °C in order to produce a first mixture. A gelling agent and water are also provided and heated up to, at least, 80 °C in order to produce a second mixture. By combining the first and second mixtures, a thick suspension is produced, which is heated up to, at least, 112 °C, to produce a gum base. This gum base is cooled down to an optimal temperature, below 90 °C, for adding an active substance and said active substance is substantially mixed, in a uniform manner, with the gum base. In some embodiments, the active substance is a drug that is mixed with an edible oil and added to said thick suspension after it has cooled. The gum base and the mixture of the active substance are then poured into molds and left to cure. Also disclosed in this document is the process of manufacturing the therapeutic edible gum described, which can comprise active substances such as ibuprofen, among others.

The present pharmaceutical formulation differs from what is disclosed in the two previous documents because, just like other similar traditional formulations meant to provide easier swallowing of medication, do not take into account the advantages of using a natural and healthy excipient, containing ingredients such as sugars that contribute to many health problems (caries, overweight, diabetes, others), missing the purpose of the excipient comprising only or mainly ingredients that do not have interference with general health condition of the consumer or patient. The excipient for this formulation is a sweet gelatin or jelly (one of the common types of excipients) and not a healthy and natural sweet. It also regards the preparation of a specific excipient which does not comply with the requirements for healthy and natural products. Unlike the previous disclosed, the present pharmaceutical formulation provides the masking of the unpleasant taste of the active substance(s), because the used healthy and natural as the basis for the introduction of those active substances have strong aromas, e.g. fruit, through the inclusion of a large amount of fruit or fruit concentrate in its composition, and are naturally sweetened, without resort to synthetic or other sugars, harmful to both humans and animals. This pharmaceutical formulation facilitates the administration of medication to children without the need to add sugars and/or sweeteners which can be harmful - comprising instead natural sweeteners, such as fructose, maltitol syrup or stevia, among others - and/or artificial flavoring agents used in traditional formulations of medicines and that contribute to the generation of dental caries in more than half of the children who ingest medications and also contributing to increase the incidence rate for overweight and diabetes. It is provided a healthier drug presentation, more appealing to children and elders, very easy to administrate, which until now was not possible to achieve by any of the products available on the market, presenting itself as the best solution to the main issues pointed out by the patients who have to ingest medication, which had no appropriate and comfortable solution until now, or the available solution implied negative consequences for the patient or consumer.

In short, one of the provided solutions according to the present application, in addition to the formulation of active substances and medicaments comprising such formulations in a presentation or dosage form which are easy to swallow, easy to administer, with pleasant taste, easy to mix and easy to carry, is also the fact that it is harmless to the health condition of patients due to the nature of its ingredients and compositions. The technical problem "How to formulate active substances and medicaments comprising them, in a natural and healthy dosage form, without resorting to synthetic sugars and artificial additives?" is answered by the present application, unlike the previous disclosures and similar products, which constitute the state of the art.

### General Description

The drugs or medicaments administered orally are administered to the patient under various presentation or dosage forms, such as liquid solutions, emulsions or suspensions, in solid form as capsules or tablets (any solid moulded or compressed dosage form, including oblong pills), among other known dosage forms in the art.

The drugs administered in the form of tablets or capsules are usually considered as being fully ingested. Thus, the often unpleasant taste of the active substance is not taken into account in medical formulations, unless the purpose is to provide a means to prevent the taste is noticed during the short period of time in which the medicament remains in the mouth. Such means may include a thin coating and of rapid dissolution, or customizing the use of gelatin or jelly capsules (the outer layer of gelatin capsule maintains the active substance inside until the capsule has been swallowed), or simply a drug or medicament firmly compressed in a way that it doesn't start to disintegrate during the short period of time in which it is supposed to stay in the mouth.

Children and the elder, among other people, or animals, have difficulty in swallowing the whole dosage forms of medicaments, regardless of its formulation, whether by its flavor, size, rigidity, period of dissolution, etc.

Thus, in those cases where the dosage to be administered cannot be produced in a ready to use form, such as very small tablets or capsules, it is desirable to provide a liquid presentation form or a soluble form, in addition to tablets or capsules that are designed to be swallowed whole. Even when the pharmaceutical formulation can be formulated as a liquid it is also desirable to provide a solid form because it is usually more convenient, light and less fragile regarding transportation purposes.

A common issue with liquid or soluble forms of medication is their flavor, often unpleasant, and associated to the active substance, which is perceived by the consumer/patient during the period of time that it contacts with the mouth. Frequently, the taste of the active substance in a medicament is controlled and masked by adding flavoring ingredients to the formulation. For example, in the case of aspirin for children where the dosage is small enough so that the amount of flavoring agents required to mask the taste of medicinal formulation is also low, not requiring a big dosage form for the medicament. However, this flavor masking is usually achieved by the use of sugars and/or other harmful ingredients to the consumer's health condition.

The esophagus is a muscular tube that carries food from the mouth to the stomach, being located mainly at the level of the human's chest. Normally we don't perceive this organ, except when we swallow. However, if the internal coating (called mucosa) of the esophagus becomes inflamed, we may feel chest pain or difficulty in swallowing. This inflammation of the esophagus is called esophagitis.

Some common medication can cause chemical burn of the esophagus. Pills that have a greater probability to cause esophagitis include: aspirin, doxycycline, iron supplements, non-steroidal anti-inflammatory drugs, such as ibuprofen or naproxen, medicines for osteoporosis, such as alendronate or risedronate.

Dysphagia is a term that refers to the difficulty in swallowing (oropharyngeal dysphagia) and the sensation of solid food and/or liquids "stuck" in the throat or esophagus (esophageal dysphagia). This condition causes discomfort and difficulty in chewing and swallowing, which can lead to poor nutrition and also dehydration or in the case of the need of medication intake, this condition may impair the treatment and therapeutics. This is a worldwide problem that affects approximately 60% of the population in developed countries, especially the children and elderly.

Resisting and/or crying is the means used by many children to reject medication, which is normal, given its unpleasant taste or inadequate form of presentation, challenging both parents and doctors, and compromising the success of treatments. To aid the administration of drugs, in general, pharmaceutical companies chose the simplest way available: if the taste is bitter, just add sugar to make it more pleasant and easier to ingest. But this solution also brings negative consequences, since a large part of pediatric medicines contain an excessive amount of sugar (between 60% to 80 %), some with more than 80% of total weight or volume, contributing to generating caries and also for the excess weight and diabetes in more than 50% of the children who take these medicines. One would not expect that a medicament could be so harmful. A child rarely likes medicines, but the majority of children often like sweets. The solution is to produce gum, lollipops, candy, drops, lozenges, chewing-gum and chocolates, among other sweets, comprising up to 100% natural and healthy ingredients, among other adaptable formulations, without synthetic sugars, lactose-free, gluten-free, without coloring agents or artificial preserving agents and adding them active substances for obtaining stable formulations to be comprised by medicaments to be easily administered to humans and animals, without the drawback of high sugar level in regular medicaments.

The adopted definition of an ingredient is any substance, including additives, used in the manufacture or preparation of formulations and drugs or medicaments according to the present application, which remains in the final product, unchanged or in a modified form.

"Natural Ingredients" are usually foods that are minimally processed, or do not contain food additives or do not contain other additives, such as hormones, antibiotics, sweeteners, coloring agents, flavoring agents, and others which are not found in food in its original composition. The Codex Alimentarius from Food and Agriculture Organization of the United Nations (FAO) does not recognize the term "natural" but has a definition for organic food. A natural ingredient is that which is organic, that is, originates from conventional agriculture, such as fruit, vegetables and/or originating from animals and that have no harmful effect in a human or animal body.

A natural product is a mixture of several natural ingredients.

A harmless ingredient is that which does not impairs the health of its consumer/user, that does not cause harm; harmless and not dangerous.

An active substance is any substance with pharmacological activity or other direct effect in the diagnosis, such as: cure, relief, treatment or prevention of diseases, clinical conditions and their symptoms; or that could affect any function of the human body.

The present pharmaceutical formulation is intended to incorporate any active substance applicable and stable in ingestible formulations for administration to humans and animals, such as paracetamol, ibuprofen, acetylsalicylic acid, among other substances for analgesics, antipyretics, anti-conception, anti-inflammatory, anti-allergic, muscle relaxants, diuretics, etc.

A formulation or pharmaceutical dosage form is the final state presented by active substances after being submitted to the necessary pharmaceutical operations in order to facilitate its administration and optimize the desired therapeutic effect. The subjection of the active substances to pharmaceutical operations is necessary due to the fact that the majority of the active substances cannot be directly administered to the patient.

The pharmaceutical operations involve all preparations and implemented actions with the purpose of transforming an active substance, also called a drug or active principle, in a pharmaceutical form. In some cases only one single operation is used, however, for the obtaining of a pharmaceutical form, several pharmaceutical operations are frequently implemented, namely:
- Weighing
- Grinding
- Dissolution in appropriate solvent
- Incorporation
- Homogenization of the mixture
- Packaging
- Etc.

Medicament is a pharmaceutical product, technically obtained or prepared, with prophylactic, curative, palliative or for diagnostic purposes. Medicaments comprising the formulations of the present application may be presented in the following forms:
- tablets
- capsules
- dragees
- pills
- solution
- suspension
- emulsion
- ovules
- ointments
- suppositories
- liniments

The casing of capsules may usually comprise starch or gelatin.

The choice of pharmaceutical formulation depends mainly:
- of the physical and chemical nature of the active substance;
- of its action mechanism;
- of the target site of the drug or medicament.
- of the dosage - amount of active substance in the pharmaceutical form.

An excipient is defined as a pharmacologically inactive substance, used as a vehicle for carrying the active substance. In the formulation, it can act as a binding agent, disintegrating agent, ligand, lubricant, surfactant, solubilizing agent, suspension agent, thickening agent, diluent, emulsifier, stabilizer, preservative, coloring agent, flavoring agent.

The present application refers to the formulation of medicines based on healthy, natural, gluten-free, lactose-free excipients without coloring agents or artificial preservatives.

Medicaments comprise mainly two elements, the active substance (that cures or attenuates the symptom(s) or the disease) and the excipient (which embodies the active substance). In the present application the traditional excipient of a given medicament is replaced by healthy and natural sweet tasting elements (chocolates, lozenges, sweets, gum, among others). The manufacturing method of such formulations and medicaments should always be adapted and arranged to fit the optimal conditions of manufacturing the active substance and the selected excipient, by at least one of two main methods: inclusion on pre-production and inclusion on co-production. The inclusion on pre-production, in a first phase, the sweet product/candy (whilst excipient) is completely produced, fulfilling the requirements of natural and healthy food and in the second phase the active substance is introduced (e.g. by injection within the excipient, compression, or by other means).

On inclusion by co-production, during phase 1 the sweet product (whilst excipient) is produced but not into its final presentation form (being kept in the liquid state, without solidification), complying with the requirements of natural and healthy, and in the second phase the active substance is introduced (by direct adding) and on a third phase, the excipient is solidified.

During inclusion on co-production, it is also possible to add the active substance during the manufacture stage of the sweet product (whilst excipient) together with the remaining ingredients comprised by the excipient.

The present disclosure regarding manufacturing methods are not limiting, other methods may be used, such as traditional methods of introducing active substances in excipients, or other useful methods which are also effective and easy to implement and perform, once they are optimized.

### Scope of Use

For example, each healthy chocolate generates, on average, 25 calories, has no contraindications and even diabetics can eat it, because it does not contain sugar, or lactose, comprises more than 50% of cocoa, soya flour and is sweetened with naturally sweet plant extracts (without sugar), rendering it a good excipient for medicaments such as, for example, those which are based on iron (Fe), magnesium (Mg) or phosphorus (P).

Lollipops are an additional vehicle for drug administration. The sweet product dissolves slowly while in the mouth, releasing the drug for absorption. Lollipop with glutamine, for example, accelerates healing of aphthas and aphthous stomatitis. This pharmaceutical form is also particularly indicated for patients with swallowing difficulties, such as the elderly or cancer patients under treatment. Due to the a pleasant appearance and taste, this pharmaceutical form increases the patient's adherence to the treatment.

Gums are naturally flavored with fruit in order to minimize the characteristic unpleasant flavors of medicament's formulation components, and are capable of incorporating a great volume of substances. They are also good options for children who are over-weight, providing them with a large variety of flavors in order to render them more attractive.

The present application provides medicament production in the form of "sweet products" (chocolates, candy, lozenges, gum, etc.) with pleasant flavor, such as fruit, cocoa, and others. This is an easier way to administer medication to human and animal patients, therefore allowing a more effective dosage routine and being less laborious for people who administrate medication.

### Detailed Description

### Method

The method of manufacturing the formulation of the present application is characterized by the addition of at least one active substance to the excipient in one or more stages of production of such excipient.

In a preferred embodiment, at least one active substance is added to the excipient in an initial stage of production of the latter, resulting in a mixture that is subjected to one or more pharmaceutical operations for obtaining the formulation of the present application.

According to another preferred, at least one active substance is added to the excipient in an intermediate stage of production of the latter, in that (i) in a first step the excipient is produced and is in liquid state; (ii) in an additional step, an active substance is added to the excipient; and (iii) in an additional step, the formulation is solidified.

According to another preferred embodiment, at least one active substance is added to the excipient in final stage of production of the latter, in that (i) in a first step the excipient is produced in its entirety; (ii) in an additional step, an active substance is added to the excipient in its final presentation form, whether it is solid, liquid, gas or gel, or other presentation form known in the art.

According to a preferred embodiment, a medicament is produced and used for the treatment and relief of symptoms associated with a given disease, comprising or consisting of a formulation in accordance with the formulation of the present application, by means of the incorporation of the method of production of said formulation in the methods commonly known for the manufacture of medicaments and pharmaceutical formulations therein.

### Formulations

### Examples

The examples herein described are mere embodiments, not limiting the scope of the present application, which respects to the integration with any method of production of formulations and medicaments, comprising any active substance.

### Example of formulation of healthy and natural gum for incorporating and adding active principles:

Characteristics - Sweet products, providing low calory income and not contributing to developing dental caries, obesity or diabetes.

Ingredients: maltitol syrup, gelatin, acidulant (citric acid), concentrated fruit juices (20%), acerola powder, natural flavorings, dyes (nettle, spinach extract, turmeric extract, currant, carrot, pumpkin and apple concentrate), vegetable oil, coating (beeswax and carnauba).

### Nutritional Information (per 100g)

Protein 6.1g
Carbohydrates 75g
Sugars 0g
Polyols 75g
Starch 0g
Lipids 0.1g
Saturated 0.1g
Fiber 0g
Sodium 0g
Vitamin C (acerola) 50mg (63% DDR)

### Example regarding the preparation of healthy and natural chocolate as excipient for inclusion of the active substance

500g cocoa powder
400g soy flour
2 eggs or 1 apple, or 1 banana
3g of stevia extract or 30g of stevia leaves

The amounts employed may vary, depending on the intended texture, flavor, flexibility and hardness required, in accordance with the methodologies commonly used in the art.

Vanilla or cinnamon may be added as a sweetener and/or flavouring agent.

Mix all the ingredients and heat such a mixture until approximately 60 °C stirring permanently until the desired texture is obtained. Temperature of the mixture may be lower or higher than 60 °C, depending on the intended result.

The resulting slurry is poured into fillers or moulds of variable dimensions and shapes, for example with a capacity up to 5g, and the active substance is added thereto. To each 5g of this slurry, up to about 2 g of the active substance may be added.

### Example for Preparation of Paracetamol for subsequent inclusion in an excipient according to the present application

Pure paracetamol is sieved through a sieve mesh size 16 in a suitable mill and granulated with appropriate quantities of deionized water to form a medium/heavy granule. The sediment was dried in an suitable oven 45, until the water content was < 1%. The resulting dry granulate was then sieved through a sieve mesh size 12 to produce white granules.

## Claims

1. A pharmaceutical formulation **characterized in that** it comprises at least one active substance and a pharmaceutically effective and acceptable excipient, comprising natural and/or innocuous ingredients.

2. The pharmaceutical formulation according to the previous claim, **characterized in that** it comprises gum, lollipops, sweets, tablets, lozenges, chocolates, pills, capsules, gels, elixirs and syrups.

3. The pharmaceutical formulation according to the previous claims, **characterized in that** the excipient is a gum and comprises sweetener, gelatine or jelly, acidulant, fruit concentrate, coloring agents, vegetable oil, coating agents and vitamins.

4. The pharmaceutical formulation according to the previous claims, **characterized in that** the excipient is chocolate.

5. The pharmaceutical formulation according to the previous claims, **characterized in that** the sweetener is maltitol syrup.

6. The pharmaceutical formulation according to the previous claims, **characterized in that** the sweetener is stevia.

7. The pharmaceutical formulation according to the previous claims, **characterized in that** the acidulant is citric acid.

8. The pharmaceutical formulation according to the previous claims, **characterized in that** the coloring agents are selected from nettle, spinach extract, turmeric extract, currant, carrot, pumpkin and apple concentrate.

9. The pharmaceutical formulation according to the previous claims, **characterized in that** the coating is selected from beeswax and carnauba.

10. The pharmaceutical formulation according to the previous claims, **characterized in that** each 100 g of said formulation comprises:
| | |
|---|---|
| Protein | 6,1g |
| Carbohydrates | 75g |
| Sugars | 0g |
| Polyols | 75g |
| Starch | 0g |
| Lipids | 0,1g |
| Saturated | 0,1g |
| Fiber | 0g |
| Sodium | 0g |
| Vitamin C (acerola) | 50mg (63% DDR). |

11. A medicament **characterized in that** it comprises the formulation according to the previous claims.

12. A method of manufacturing the formulation as described in the claims 1 to 10 **characterized in that** the addition of, at least one, active substance to the excipient occurs in, at least one, stage of production of said excipient and the degree of dissolution of said formulation is variable, according to the desired level and time of absorption for each active substance, therefore varying the quantity of each of the ingredients comprised by the excipient.

13. The method according to claim 12, **characterized in that** the, at least one, active substance is added to the excipient in the initial stage of production of the latter, resulting in a mixture that is submitted to pharmaceutical operations for obtaining the formulation described in the claims 1 to 8.

14. The method according to claims 12 and 13, **characterized in that** the, at least one, active substance is added to the excipient in an intermediate stage of the production of the latter, wherein (i) in a first step the said excipient is produced and it is liquid; (ii) in an additional step, an active substance is added to the excipient; and (iii) in an additional step, the formulation described in the claims 1 to 8 is solidified.

15. The method according to claims 12 to 14, **characterized in that** the, at least one, active substance is added to the excipient in a final stage of the production of the latter, wherein (i) in a first step, said excipient is produced in its entirety; and (ii) in an additional step, an active substance is added to the excipient, in its final presentation form, solid, liquid, gas or gel.

16. A method of manufacturing a medicament as described in the claim 11 **characterized in that** comprises a formulation as described in the claims 1 to 8 by incorporation of the method as described in the claims 12 to 15.

17. The Pharmaceutical formulation as described in the previous claims 1 to 10, for use in treatment of diseases and other clinical disorders in humans and animals, **characterized in that** it is administered for the treatment and relief of symptoms caused by said diseases and clinical disorders.

18. The medicament as described in the previous claim 11, for use in treatment of diseases and other clinical disorders in humans and animals, **characterized in that** it is administered for the treatment and relief of symptoms caused by said diseases and clinical disorders.
